# EUROPÄISCHE PATENTANMELDUNG

(11) **EP 0 849 264 A1**
(43) Veröffentlichungstag der Anmeldung: **24.06.1998**
(21) Anmeldenummer: 96120601.8
(22) Anmeldetag: 20.12.1996
(51) Int. Cl.: C07D 405/14, A61K 31/505

(54) **Neue polymorphe Form von doxazosinmesylat (Form I)**

(71) Anmelder: HEUMANN PHARMA GmbH, D-90478 Nürnberg (DE)
(72) Erfinder: Grafe, Ingomar Dr. Dipl.-Chem., 90482 Nürnberg (DE); Mörsdorf, Johann Peter Dr. Dipl.-Chem., 90579 Langenzenn (DE)
(74) Vertreter: Kraus, Walter, Dr.

(57) **Zusammenfassung**

Beschrieben wird eine neue kristalline und wasserfreie Form von Doxazosin-Mesylat. Die neue Form ist kristallin und wasserfrei und durch die folgenden Reflexlagen hoher und mittlerer Intensität
- 15,40°: 24,15°
- 16,85°: 25,81°
- 18,06°:
charakterisiert. Die erfindungsgemäße neue Form von Doxazosin-Mesylat weist aufgrund ihrer kristallinen Eigenschaften überraschende Vorteile sowohl hinsichtlich ihrer Synthese als auch für die galenische Verarbeitung zu festen Arzneiformen auf. Beschrieben werden weiterhin ein Verfahren zur Herstellung der neuen Form sowie Arzneimittel, die die neue Form von Doxazosin-Mesylat enthalten.

## Beschreibung

Die Erfindung betrifft eine neue, kristalline und wasserfreie Form von Doxazosin-Mesylat, ein Verfahren zu ihrer Herstellung und diese neue Form I enthaltende Arzneimittel.

1-(4-Amino-6,7-dimethoxy-2-chinazolyl)-4-[(2,3-dihydro-1,4-benzodioxin-2-yl)carbonyl]piperazinmethansulfonat mit dem INN-Namen Doxazosin-Mesylat ist ein Diaminochinazolinderivat aus der Klasse der α₁-Rezeptorenblocker. Es zeigt eine große strukturelle Ähnlichkeit zu den älteren Vertretern dieser Klasse, Prazosin-Hydrochlorid und Terazosin-Hydrochlorid. Während die beiden letztgenannten Wirkstoffe primär nur bei der Behandlung des Bluthochdrucks eingesetzt werden, eröffnet sich im Falle des Doxazosin-Mesylats eine weitere Indikation, nämlich die Behandlung der benignen Prostatahyperplasie.

Im Gegensatz zu Prazosin und Terazosin wird Doxazosin therapeutisch nicht als Hydrochlorid eingesetzt, sondern als Mesylat, d.h. als Salz der Methansulfonsäure.

Trotzdem bereits Doxazosin-Mesylat-enthaltende Arzneimittel im Handel sind, ist das Doxazosin-Mesylat bislang noch nicht beschrieben worden. Auch die US-PS 4 188 390, die erstmals Doxazosin offenbart, enthält keine Beschreibung von Doxazosin-Mesylat. In den Beispielen dieser Druckschrift wird lediglich das Doxazosin-Monohydrochlorid beschrieben.

Das Hydrochlorid ist jedoch wegen seiner extremen Schwerlöslichkeit in Wasser für pharmazeutische Zwecke ungeeignet.

Versuche zur Herstellung von Doxazosin-Mesylat auf den üblichen Wegen gestalten sich sehr schwierig und führen zu unbefriedigenden Ergebnissen. Einerseits ist Doxazosin-Base in den für Salzbildungen gängigen Lösungsmitteln schwer löslich. Lediglich in polaren, aprotischen, hochsiedenden Lösungsmitteln, wie beispielsweise Dimethylformamid, ist sie ausreichend löslich. Jedoch ist in diesen Lösungsmitteln die Löslichkeit des Doxazosin-Mesylats ähnlich der der Base, so daß die zu erhaltenden Ausbeuten an Mesylat absolut unbefriedigend sind. Außerdem ist Dimethylformamid aus pharmakologischer Sicht ein kritisches Restlösungsmittel in Arzneimittelwirkstoffen.

Andererseits kann Doxazosin-Base in schwachen Säuren, wie z.B. Essigsäure, gelöst werden und durch Zugabe von Methansulfonsäure das Mesylat gefällt werden. Jedoch erhält man bei dieser Arbeitsweise bei Raumtemperatur ein unfiltrierbares Gel. Arbeitet man bei höheren Temperaturen, beispielsweise 50°C, so klumpt dieses Gel zusammen oder ölt bei höheren Konzentrationen als zweite, nicht erstarrende Phase aus. Durch Zusatz organischer Lösungsmittel, beispielsweise Aceton, kann die Absaugfähigkeit des gefällten Doxazosin-Mesylats zwar verbessert werden, doch führt die Trocknung dieses Produkts zur Bildung von Klumpen aufgrund des hohen Feuchtegehalts. Letztendlich erhält man eine amorphe und hygroskopische Form des Doxazosin-Mesylats.

Der Erfindung liegt daher die Aufgabe zugrunde, eine kristalline und wasserfreie Form des Doxazosin-Mesylats bereitzustellen, die aufgrund ihrer physikalischen Eigenschaften, insbesondere ihrer Kristalleigenschaften und ihres Verhaltens gegen Wasser, sowohl in ihrer chemischen Herstellung als auch in der galenischen Formulierung gut zu handhaben ist.

Diese Aufgabe wird erfindungsgemäß durch eine neue, kristalline und wasserfreie Form des Doxazosin-Mesylats gelöst, die nachstehend als Form I bezeichnet wird.

Gegenstand der Erfindung ist daher die Form I von Doxazosin-Mesylat, die dadurch gekennzeichnet ist, daß sie ein Röntgen-Pulverdiagramm mit den folgenden Reflexlagen hoher und mittlerer Intensität:
- 15,40°: 24,15°
- 16,85°: 25,81°
- 18,06°:
aufweist und daß sie kristallin und wasserfrei ist.

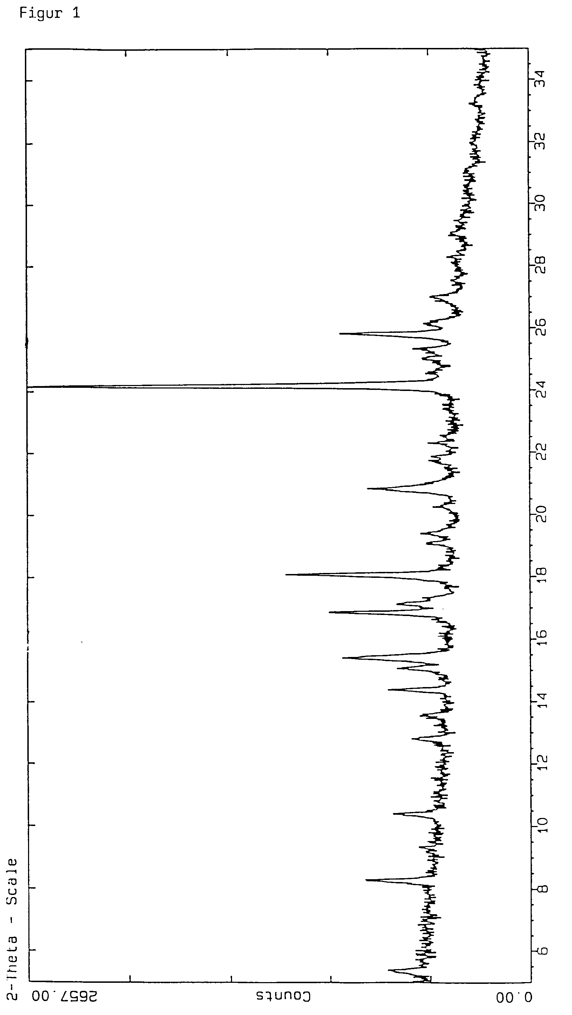

Die erfindungsgemäße Form I ist durch ein Röntgendiffraktogramm, gemessen unter Verwendung von Cu-K_{α1}-Strahlung und eines Ge-Monochromators mit einer Schrittweite von 0,017° im Beugungswinkelbereich von 2 θ = 5-35°, das in Figur 1 wiedergegeben ist und folgende Reflexlagen hoher und mittlerer Intensität aufweist, charakterisiert:
- 15,40°: 24,15°
- 16,85°: 25,81°
- 18,06°:

Die erfindungsgemäße Form I von Doxazosin-Mesylat unterscheidet sich über das Röntgendiffraktogramm hinaus noch in einer Reihe von weiteren Eigenschaften von den anderen Formen des Doxazosin-Mesylats. Diese Eigenschaften können daher ebenfalls zur Unterscheidung von den anderen Formen herangezogen werden.

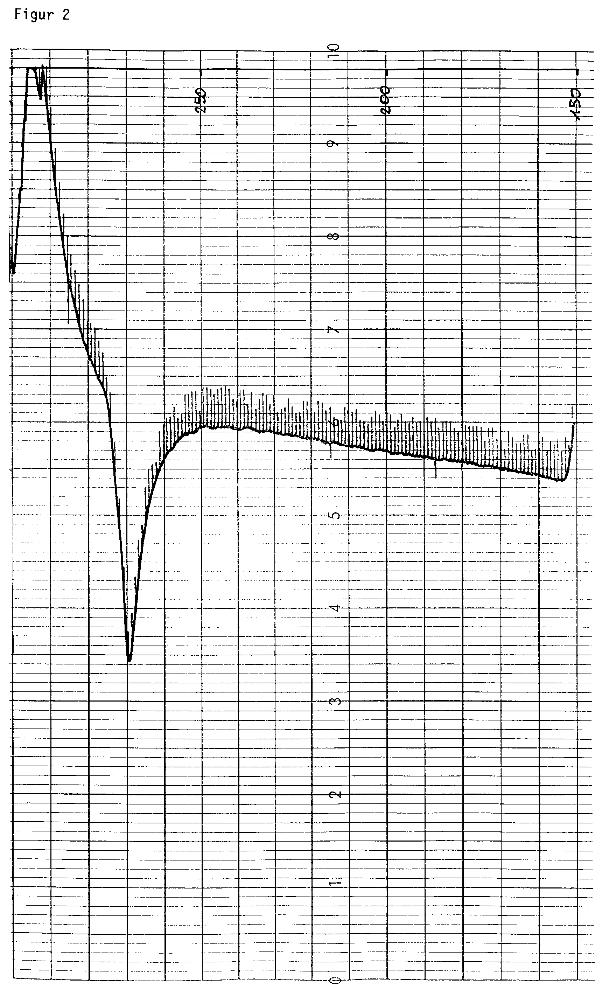

Eine weitere Charakterisierung der Form I des Doxazosin-Mesylats kann mit Hilfe der Differentialthermoanalyse (DTA) erfolgen. Gemäß dem in Figur 2 dargestellten DTA-Spektrum der Form I mit einem Meßbereich von 150 bis 300°C ist die Form I durch einen einzigen, endothermen Peak bei 275°C gekennzeichnet, der dem Schmelzpunkt der Form I entspricht.

Ein weiterer Gegenstand der Erfindung ist ein Verfahren zur Herstellung der oben beschriebenen, erfindungsgemäßen Form I von Doxazosin-Mesylat, das dadurch gekennzeichnet ist, daß man
(1) Doxazosinbase in einem Alkohol-Wasser-Gemisch suspendiert,
(2) die Base durch Zugabe einer schwachen Säure in das lösliche Salz der schwachen Säure umwandelt,
(3) das Doxazosin-Mesylat durch Zugabe von Methansulfonsäure und Einstellen des pH-Werts durch Zugabe einer Base auf einen Wert im Bereich von 2 bis 4 ausfällt und daß man
(4) das ausgefällte Doxazosin-Mesylat, gegebenenfalls nach Rühren und Abkühlen durch Filtrieren, Waschen mit einem organischen Lösungsmittel und Trocknen, gewinnt.

In der ersten Stufe des erfindungsgemäßen Verfahrens wird Doxazosin-Base in einem Alkohol-Wasser-Gemisch suspendiert. Geeignete Alkohole sind beispielsweise niedere Alkylalkohole, wie Methanol, Ethanol oder n-Butanol, sowie Alkylglykole, wie z.B. Methylglykole. In dem Alkohol-Wasser-Gemisch beträgt das Volumenverhältnis von Alkohol :

Wasser 95:5 bis 50:50, vorzugsweise 80:20 bis 90:10. Die Suspendierung erfolgt vorzugsweise unter Rühren und bei einer Temperatur von 40°C bis zur Rückflußtemperatur des verwendeten Alkohol-Wassergemisches.

In der zweiten Stufe des erfindungsgemäßen Verfahrens wird die Base durch Zugabe einer schwachen Säure in das lösliche Salz dieser Säure umgewandelt. Beispiele für die schwache Säure sind Ameisensäure, Essigsäure oder Milchsäure. Die schwache Säure wird vorzugsweise im Überschuß (bis fünffach) zu der Doxazosin-Base zugegeben.

In der dritten Stufe des erfindungsgemäßen Verfahrens wird das Doxazosin-Mesylat durch Zugabe von Methansulfonsäure und Einstellen des pH-Werts durch Zugabe einer Base auf einen Wert im Bereich von 2 bis 4 ausgefällt. Das Verhältnis von Doxazosin-Base zu Methansulfonsäure liegt im Bereich von 1:1 bis 1:1,1 und ist vorzugsweise äquimolar. Die Methansulfonsäure wird vorzugsweise als 70%ige wäßrige Lösung eingesetzt. Durch Zugabe einer Base, vorzugsweise von konzentriertem Ammoniak, wird der pH-Wert auf einen Wert im Bereich von 2 bis 4, vorzugsweise von 2,5 bis 3, eingestellt. Die Reaktionstemperatur in dieser Stufe ist 20 bis 60°C, vorzugsweise 20 bis 40°C.

Die Einhaltung des wie oben definierten pH-Bereichs ist deswegen wesentlich, um einerseits eine Hydrolyse der Carbonamidgruppe des Doxazosins und andererseits eine Mitfallung der Doxazosin-Base zu verhindern.

In der letzten Stufe des erfindungsgemäßen Verfahrens wird das ausgefällte Doxazosin-Mesylat, gegebenenfalls nach Rühren und Abkühlen durch Filtrieren, Waschen mit einem organischen Lösungsmittel und Trocknen, gewonnen. Vorzugsweise wird das ausgefällte Doxazosin-Mesylat 4 bis 8 Stunden, vorzugsweise 6 Stunden, bei einer Temperatur von 20 bis 60°C, vorzugsweise 40°C, gerührt. Gegebenenfalls wird vor dem vorzugsweise durch Absaugen erfolgenden Filtrieren auf eine Temperatur von 10 bis 30°C, vorzugsweise 20°C, abgekühlt. Das erhaltene Produkt wird mit einem organischen Lösungsmittel, vorzugsweise einem niederen Alkylalkohol, wie Methanol, gewaschen und im Vakuum getrocknet.

Die erfindungsgemäße Form I des Doxazosin-Mesylats hat aufgrund ihrer kristallinen Eigenschaften überraschende Vorteile sowohl im Hinblick auf ihre Synthese und die Produktreinheit als auch für ihre galenische Verarbeitung zu festen Arzneiformen. Wie oben beschrieben, fallen die auf den üblichen Wegen erhaltenen Formen des Doxazosin-Mesylats in Form gelartiger, auch in Gegenwart von organischen Lösungsmitteln sehr voluminöser Niederschläge an, die große Mengen Mutterlaugen einschließen und daher Feuchtegehalte bzw. Trockenverluste von bis zu 50% aufweisen. Dadurch werden im getrockneten Produkt Verunreinigungen, insbesondere färbende Verunreinigungen, eingeschlossen bzw. adsorbiert. Desweiteren resultieren durch das gelartige voluminöse Produkt extrem lange Filtrations- bzw. Zentrifugationszeiten, die vom Prozeßstandpunkt her sehr nachteilig sind.

Dagegen wird die erfindungsgemäße Form I in Form eines gut kristallinen, farblosen und problemlos filtrier- bzw. zentrifugierbaren Feststoffs erhalten. Anhaftende Mutterlauge läßt sich durch Waschen des Filterkuchens mit einem geeigneten Lösungsmittel problemlos entfernen, so daß ein Produkt hoher Reinheit erhalten wird.

Amorphe Feststoffe, und erst recht hygroskopische Feststoffe, sind in der galenischen Verarbeitung sehr schlecht zu verarbeiten, da sie beispielsweise geringe Schüttdichten und mangelhafte Fließeigenschaften aufweisen. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehalts oder der Stabilität in den produzierten Fertigarzneimitteln, zu erhalten.

Die erfindungsgemäße Form I des Doxazosin-Mesylats ist therapeutisch in der gleichen Weise anwendbar wie die Doxazosin-Base und ihre pharmazeutisch annehmbaren Säureadditionssalze, wie das im Handel befindliche Doxazosin-Mesylat mit nicht bekannten morphologischen Eigenschaften. Hauptindikationsgebiete sind die Behandlung des Bluthochdrucks sowie die Behandlung der benignen Prostatahyperplasie.

Ein weiterer Gegenstand der Erfindung ist daher ein Arzneimittel, das dadurch gekennzeichnet ist, daß es neben üblichen Hilfs- und Trägerstoffen die Form I von Doxazosin-Mesylat enthält.

So kann die erfindungsgemäße Form I des Doxazosin-Mesylats zu den üblichen Darreichungsformen mit Einschluß von peroralen und parenteralen Darreichungsformen formuliert werden. Bevorzugte Formulierungen sind Tabletten oder Kapseln. Sie können durch herkömmliche Mischverfahren und unter Anwendung herkömmlicher Hilfs- und Trägerstoffe, wie Bindemittel, Sprengmittel, Aromatisierungsmittel und dergleichen, hergestellt werden. Die Dosierung entspricht bekannten Formen von Doxazosin-Salzen.

Das nachstehende Beispiel erläutert die Erfindung.

### Beispiel

### Herstellung der erfindungsgemäßen Form I von Doxazosin-Mesylat

112,5 g Doxazosin-Base werden bei 60°C in einem Gemisch aus 140 ml Wasser und 500 ml n-Butanol durch Zugabe von ca. 33 ml 85%iger Ameisensäure gelöst. Zur Lösung werden anschließend langsam 27 ml Methansulfonsäure (70%) gegeben. Dann wird durch Zugabe von konzentriertem Ammoniak ein pH-Wert von 3 eingestellt.

Bei 40°C fällt langsam ein feiner Niederschlag, der nach ca. 6 Stunden Rührzeit bei 40°C und Kühlen auf 20°C abgesaugt wird.

Nach Waschen mit Methanol und Trocknung im Vakuum erhält man 116 g (85% d. Th.) eines farblosen Feststoffs der Form I, der das in Figur 1 gezeigte Röntgendiffraktogramm und das in Figur 2 gezeigte DTA-Spektrum aufweist.

## Patentansprüche

1. Form I von Doxazosin-Mesylat, dadurch gekennzeichnet, daR sie ein Röntgen-Pulverdiagramm mit den folgenden Reflexlagen hoher und mittlerer Intensität:
15,40° 24,15°
16,85° 25,81°
18,06°
aufweist und daß sie kristallin und wasserfrei ist.

2. Verfahren zur Herstellung der Form I von Doxazosin-Mesylat nach Anspruch 1, dadurch gekennzeichnet, daß man
(1) Doxazosinbase in einem Alkohol-Wasser-Gemisch suspendiert,
(2) die Base durch Zugabe einer schwachen Säure in das lösliche Salz der schwachen Säure umwandelt,
(3) das Doxazosin-Mesylat durch Zugabe von Methansulfonsäure und Einstellen des pH-Werts durch Zugabe einer Base auf einen Wert im Bereich von 2 bis 4 ausfällt und daß man
(4) das ausgefällte Doxazosin-Mesylat, gegebenenfalls nach Rühren und Abkühlen durch Filtrieren, Waschen mit einem organischen Lösungsmittel und Trocknen, gewinnt.

3. Arzneimittel, dadurch gekennzeichnet, daß es neben üblichen Hilfs- und Trägerstoffen die Form I von Doxazosin-Mesylat nach Anspruch 1 enthält.
